# EUROPEAN PATENT SPECIFICATION

(11) **EP 0 422 855 B1**
(45) Date of publication and mention of the grant of the patent: **02.03.1994**
(21) Application number: 90310961.9
(22) Date of filing: 05.10.1990
(51) Int. Cl.: F04B 43/08

(54) **Two-cycle peristaltic pump**
Peristaltische Pumpe mit zwei Stufen
Pompe peristaltique à deux temps

(30) Priority: 10.10.1989 US 419193
(43) Date of publication of application: 17.04.1991
(73) Proprietor: IMED CORPORATION, San Diego California 92131 (US)
(72) Inventor: Meijer, Robert S., San Diego, California 92123 (US)
(74) Representative: Coxon, Philip

(56) References cited:
- EP-A- 0 090 440
- DE-A- 2 005 269
- US-A- 4 586 882

## Description

The present invention relates to devices and methods for pumping fluid through a resilient tube and, more particularly, pertains to fluid pumps. More specifically, the present invention pertains to peristaltic pumps which sequentially squeeze a resilient tube to force fluid through the tube. The present invention is particularly, but not exclusively, useful as a pump for the infusion of medical solutions to a patient.

Over the years there have been a number of pumps developed for infusion of medical solutions to patients. Such pumping of fluids has been routinely accomplished through a wide variety of well known pumping mechanisms. In the administration of fluids to a patient, it is desirable that the pump be of the "non-wetting" variety, such as that exemplified by the well known peristaltic pump. A peristaltic pump is a type of pump which uses wave-like motion against the walls of a flexible tube containing the fluid being pumped. The non-wetting-type pump is particularly useful in hospital and medical situations in that the fluid being pumped is not subject to contamination through direct contact with the component parts of the pump. In like fashion, if corrosive fluids are being pumped there is no direct contact of corrosive fluid with component parts of the pump.

Another desirable characteristic of pumping mechanisms in general is for the pump to deliver fluid at a rate which remains reasonably constant. In other words, throughout the pumping cycle, the rate of flow should remain even, without any surges or significant variations in the flow rate.

Peristaltic pumps of the non-wetting variety are basically one of two types, namely rotary peristaltic pumps and linear peristaltic pumps. A disadvantage of rotary peristaltic pumps, however, is that they have relatively poor efficiency. In addition, they impose high shear and tension stresses on the tubing which is used to convey the fluid. Another disadvantage is that because of the high forces typically produced by rotary peristaltic pumps, the tubing eventually experiences spalling of its inner walls. There is also, after a period of time, permanent plastic deformation, or "set", of the tubing. In other words, its normally circular cross section becomes flattened into a more oval shape.

Linear peristaltic pumps, in comparison, typically use reciprocating parts to provide peristaltic action against the flexible tube in order to move the fluid through the tube. Such peristaltic pumps consist or a plurality of reciprocating pumping fingers, typically twelve (12), that are sequentially urged against the tube to occlude adjacent segments of tubing in wave-like action. Although linear peristaltic pumps overcome some of the above-stated disadvantages associated with rotary peristaltic pumps, they do so at considerable added cost and with the greater complexity added by the mechanism needed to properly synchronise twelve (12) pumping fingers. Since the pumping fingers are urged to sequentially occlude adjacent segments of tubing, the crushing forces imposed on the tubing and fluid are comparable to those encountered with rotary peristaltic pumps. There is less damage, however,than that caused by rotary peristaltic pumps, since the occlusion forces are localised to the area beneath each finger rather than being applied in movement along the whole length of the tubing. Nonetheless, even with a linear peristaltic pump, there is still some damage such as plastic deformation of the tubing. As a consequence, the structural integrity of the tube carrying the fluid is compromised and as the tubing assumes a progressively more oval cross-sectional shape, the volume and flow rate of the fluid delivered in each pumping cycle is affected.

Furthermore, in order to smooth the pumping transition from one cycle to the next, some linear peristaltic pumps have what is called a "wrap" cycle. during a "wrap" cycle, the motor driving the pump is accelerated to quickly move the upstream finger into occlusion. Thereafter, the motor can resume normal speed to sequentially squeeze and occlude adjacent portions of the tube in its wave-like cycle action. Incorporating this "wrap" cycle can require use of a relatively large and expensive motor with high acceleration capability. Also, because fluid is not delivered during this "wrap" cycle, most linear peristaltic pumps use many fingers (e.g. twelve (12) additional pumping fingers, as mentioned earlier) to minimize the proportionate time of the "wrap" cycle. Maintaining proper alignment and relational movement between such a plurality of fingers also deteriorates the reliability of operation of the device and increases manufacturing costs.

EP-A-0090440 discloses a pump including a separate disposable pump chamber which can be connected in a fluid line. The pump chamber has three flexible rolling diaphragm pumping chambers through which the fluid is pumped in turn to provide a substantially pulse-free flow.

It is an object of the present invention to provide an improved device for pumping fluid through a resilient tube.

According to one aspect of this invention there is provided a device for pumping fluid through a resilient tube which comprises:
means for alternately squeezing said tube at a first location and at a second location, said squeezing means being adapted to partially occlude said tube at same first location to displace a fluid volume that is approximately twice the fluid volume displaced by said squeezing means when said squeezing means partially occludes said second location; said squeezing means being moved towards said tube at a progressively slower pace as said tube becomes deformed to establish a substantially uniform rate of fluid flow through said tube; and
means for alternately occluding said tube upstream from said first location and between said first and second locations, said first location being squeezed as said tube is occluded upstream from said first location, said occluding means being synchronised with said squeezing means.

According to another aspect of this invention, there is provided a device for pumping fluid through a resilient tube which comprises:
a base;
a platen mounted on said base and adapted to hold said tube;
a first pumping finger reciprocally mounted on said base to urge against said tube with movement between a withdrawn position and an extended position;
a second pumping finger reciprocally mounted on said base to urge against said tube with movement between a withdrawn position and an extended position, said first pumping finger partially occluding said tube to displace a fluid volume that is approximately twice the fluid volume displaced by the partially occluding movement of said second pumping finger;
means for moving said first pumping finger toward its said extended position as said second pumping finger is moved toward its said withdrawn position and moving said first finger toward its said withdrawn position as said second finger is moved toward its said extended position;
a first valve moulded on said base for occluding said tube upstream from said first pumping finger as said first pumping finger moves toward its extended position; and
a second valve mounted on said base for occluding said tube between said first and second pumping fingers as said second finger moves toward its extended position.

Preferably, said first pumping finger is approximately twice as large as said second pumping finger.

According to another aspect of this invention, there is provided a method for pumping fluid through a resilient tube which comprises the steps of:
alternately partially occluding said tube at a first location and at a second location, said first location being partially occluded to displace approximately twice the fluid volume displaced when said second location is partially occluded, and wherein said partially occluding step is accomplished at a progressively slower pace as said tube becomes deformed to establish a substantially uniform rate of fluid flow through said tube; and
alternately occluding said tube upstream from said first location, and between said first and second locations.

A preferred embodiment of the peristaltic pump for pumping fluid through a resilient tube comprises a base, a platen mounted on the base for holding the tube. The preferred embodiment may also comprise four (4) fingers mounted on the base engageable with the tube. The four (4) fingers preferably include, in sequence downstream, a first pinching finger, a first pumping finger, a second pinching finger, and a second pumping finger. The fingers are desirably mounted reciprocally on the base to urge against the tube between a withdrawn position and an extended position. Preferably the first pumping finger squeezes the tube to displace a fluid volume which is approximately twice the fluid volume displaced by the second pumping finger.

A drive mechanism may move the first pumping finger toward its extended position as the second pumping finger is moved toward its withdrawn position. The drive means may further move the first pumping finger toward its withdrawn position as the second pumping finger is moved toward its extended position. The first pinching finger may occlude the tube upstream from the first pumping finger as the first pumping finger moves toward its extended position. The second pinching finger may occlude the tube between the first and second pumping fingers, as the second finger moves toward its extended position.

In one embodiment, the first pumping finger displaces twice the fluid as a result of its being twice as large as the second pumping finger. In another embodiment, the first pumping finger displaces twice the fluid by travelling a distance which is approximately twice that of the distance travelled by the second pumping finger.

Also disclosed is a method for pumping fluid through the tube which comprises the steps of alternately squeezing the tube at a first and second location corresponding to the point of contact of the first and second pumping fingers. The tube is then alternately occluded or pinched at a point upstream from the first location, and at a point between the first and second locations. The tube is preferably squeezed at the first location when the tube is occluded upstream from the first location by the first pinching finger. Also, the tube is preferably squeezed at the second location while the tube is occluded between the first and second locations by the second pinching finger.

It is an advantage of the preferred embodiment of the present invention that it provides a peristaltic pump of the non-wetting type which is simple and efficient in operation. It is another advantage of the preferred embodiment that it provides a peristaltic pump which results in reduced stresses on the fluid-carrying tube and thus longer tube life. It is yet another advantage of the preferred embodiment that it provides a peristaltic pump which produces a substantially linear, and non-pulsatile flow for the fluid being pumped. Still another advantage of the preferred embodiment is that it provides a peristaltic pump which is relatively easy to manufacture, durable and reliable in its operation and comparatively cost-effective.

Reference is now made to the accompanying drawings in which:-
Figure 1 is a perspective view of the peristaltic pump in its intended environment;
Figure 2 is a side cross-sectional view of the peristaltic pump apparatus taken along the line 2-2 of Figure 1;
Figure 3 is an end cross-sectional view of the peristaltic pump apparatus taken along the line 3-3- of Figure 1;
Figure 4 is a schematic illustration of operation of the peristaltic pump shown in Figure 1;
Figure 5 is a schematic diagram illustrating the relative operation of the movement of fingers utilised in operation of the peristaltic pump in accordance with the invention; and
Figure 6 is a schematic illustration of an alternative embodiment of the peristaltic pump in accordance with the present invention.

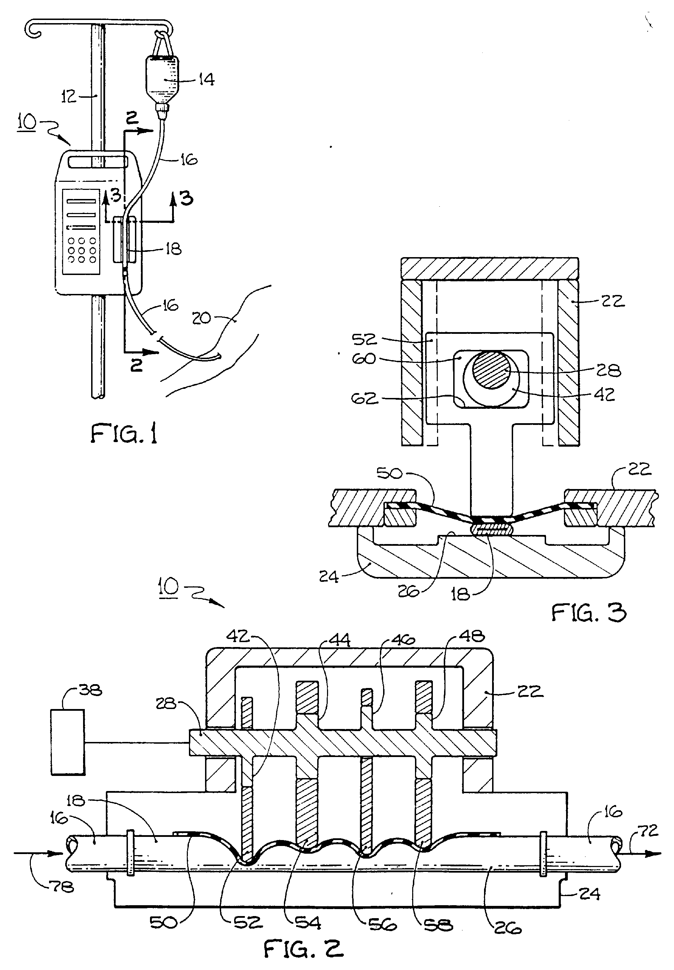

Referring now to Figure 1, there is shown a peristaltic pump apparatus, generally designated 10, shown in use in its intended environment. In particular, peristaltic pump apparatus 10 is shown attached to an intravenous (I.V.) pole 12. Attached to pole 12 is a fluid source 14 containing an I.V. fluid. Fluid source 14 is connected in fluid communication with a hollow resilient tube 16. Tube 16 is a conventional I.V. infusing-type tube for use in a hospital or medical environment, but could likewise by any type of flexible tubing, such as rubber. There is a portion 18 of flexible tube 16 which is mounted in pumping apparatus 10, for pumping fluid through tube 16 into a patient's arm 20.

The components of peristaltic pump apparatus 10 can be best appreciated now with reference to Figures 2 and 3. Peristaltic pump apparatus 10 comprises a casing 22 having a base 24 which includes a generally flat platen 26. Platen 26 provides a surface against which tube 18 may be occluded in the manner as shown in Figure 3.

Mounted in casing 22 is a rotatable shaft 28, which is driven by a motor 38. Motor 38 is a variable speed motor which provides the driving power for driving the pump shaft 28. Shaft 28 is rotatably mounted in casing 22. Shaft 28 also includes cam lobe portions 42, 44, 46 and 48.

Four (4) fingers 52, 54, 56 and 58 are reciprocally mounted in casing 22 onto base 24. Fingers 53, 54, 56 and 58 are reciprocally mounted for being urged against tube 18. To provide further protection for tube 18, and to keep dirt and other unwanted materials from the inner workings of the peristaltic pump 10, a flexible membrane 50 is connected to base 24. Each finger 52, 54, 56, 58 is reciprocally mounted to move up and down with respect to platen 26. Each finger 52, 54, 56, 58 is movable between a withdrawn position, or upper limit, and an extended position, or lower limit, to deform tube 18 a specified amount as explained hereinafter.

In particular, pinching fingers 52 and 56 are of identical configuration, and serve as pinch valves. Fingers 52 and 56 are movable between a withdrawn position as shown by finger 56 in Figure 2, and an extended position as shown by finger 52 also in Figure 2. This is also further shown with reference to Figure 3. As can be seen, rotation of shaft 28 causes corresponding rotation of cam lobe portion 42. Shaft 28 and cam lobe 42 thus rotate within orifice 60 of first pinching finger 52. Cam lobe 42 thus engages a side wall 62 of orifice 60 so as to urge pinching finger 52 to its fully extended position. This causes finger 52 to press against membrane 50 and to urge against tube 18 so as to occlude tube 18 as shown in Figures 2 and 3. These fingers are thus positioned so that the smallest possible motion of fingers 52, 56 suffices to alternately occlude or open tube 18 to allow fluid to flow beneath them. In other words, in an open or fully withdrawn position, as shown by finger 56 in Figure 2, an aperture is provided in tube 18 which is sufficient for relatively unrestricted flow of fluid beneath finger 56 at the maximum rate of the pump. Typically, the extent of the range of motion of pinching fingers 52 and 56 is fixed at no more than one (1) to three (3) times the wall thickness of the tube 18. Thus, first finger 52 and third finger 56 are essentially pinching fingers.

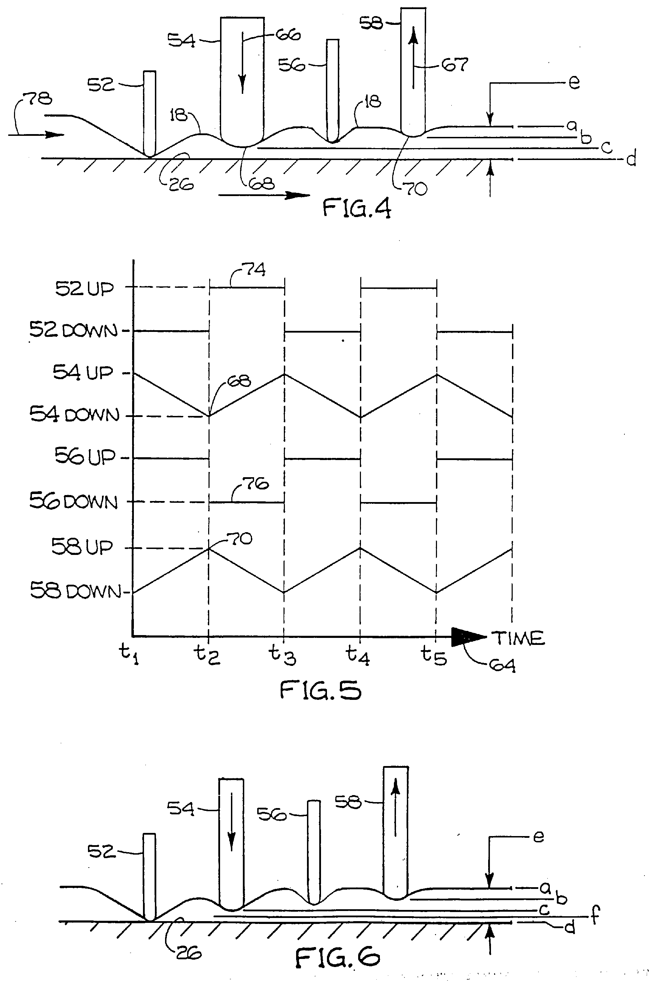

It may also be readily appreciated with reference to Figures 2 and 4 that second finger 54 and fourth finger 58 are pumping fingers in the sense that it is these fingers which squeeze tube 18 to urge fluid out of tube portion 18. In addition, first pumping finger 54 has a unique configuration and is designated as a "large" pumping finger. Finger 58 is also of a unique configuration and designated as a "small" pumping finger. With respect to the description of the invention herein, "large" and "small" describe pumping fingers which are constructed to move against tube 18 such that the amount of fluid displaced as "large" finger 54 moves downward against tube 18, is approximately two (2) times that displaced by an equal reciprocal downward motion of "small" finger 58. It is important to note that the reciprocal motion of fingers 54 and 58 is generally equal in range, but that in the fully extended position, the pumping fingers 54, 58 to not ever fully occlude the tubing. Instead, they squeeze the flexible tubing 18 from a relatively larger percent of initial tubing outside diameter to a relatively smaller percent of initial tubing outside diameter.

Operation of the present invention may perhaps be best appreciated with reference to Figures 4 and 5. In particular, Figure 4 represents relative movement of fingers 52, 54, 56 and 58. In Figure 5, the relative movement of pinching fingers 52 and 56 and pumping fingers 54 and 58 are shown in relation to one another over a period of time that includes reference points t₁, t₂, t₃, t₄ and t₅ on time line 64. As shown in Figures 4 and 5, motor 38 drives shaft 28 and cam lobe 42, 44, 46 and 48 so that fingers 52, 54, 56 and 58 are reciprocated to execute in the present embodiment a two-cycle motion as described below. The first cycle is between time t₁ and t₂, and the second cycle is between t₂ and t₃.

In the first cycle, finger 52 is held closed in its fully extended position, while finger 56 is held in its fully withdrawn or open position, as shown in Figure 4. This is indicated in Figure 5 on time line 64 at starting point time t₁. At point t₂ as shown in Figure 5, finger 52 is in the down position and finger 56 is in the up position. As the motor 38 rotates shaft 28, cam lobes 42, 44, 46 and 48 cause fingers 52 and 56 to remain in the same position, but also cause fingers 54 and 58 to move. Finger 54 moves down in the direction as shown by arrow 66 having started at upper limit or height elevation "b" and moving down to lower limit or elevation "c". Thus, in Figure 4, finger 54 is shown at its position 68, and is shown in Figure 5 where finger 54 is in a fully extended position, yet not occluding tube 18. Thus, immediately prior to t₂, finger 52 is still down. At the same time, finger 58 which started in its down position at elevation "c" at time t₁ has moved up as shown by arrow 67 to position 70 immediately prior to time t₂. Thus, finger 54 having moved downward from its extreme retracted position at elevation "b" toward platen 26 drives fluid out and toward patient 20 generally in the direction of exit arrow 72. Simultaneously, finger 58 has started moving upward from its extreme extended position, as shown in the graph on Figure 5 between time t₁ and t₂. The difference in the size of the fingers, i.e. "large" versus "small" results in a net delivery of fluid comparable to that resulting from the displacement produced by the "small" finger acting alone. In other words, since the "large" finger 54 displaced twice the volume as that of "small" finger 58, the net result of cycle one is the delivery of one unit of volume toward the exit of tube 16 as shown by arrow 72.

As cycle one is concluding at time t₂, the "large" finger 54 and "small" finger 58 have exchanged vertical positions. Also at time t₂, pinching fingers 52 and 56 exchange positions. Pinching finger 52 is raised toward its fully withdrawn position as shown at elevation point "c", and pinching finger 56 is lowered as shown by elevation line "d". Since pinching finger 56 is now closed, and pinching finger 52 is open, fluid is drawn into the tube 16, and thus, portion 18 as shown by entry arrow 78. The rate of flow of fluid into the entrance of tube 18 is twice that of the output rate, since finger 54 displaces twice as much fluid as finger 58. Then, while finger 54 is retracting between time t₂ and t₃, finger 58 is extending from elevation "b" to elevation "c" as shown in Figure 4. This produces a net delivery output of one unit of fluid via exit arrow 72.

This action progresses until time t₃ at which time cycle two has been completed. At time t₃, the pinching fingers 52, 56 again revert so that pinching finger 52 is again down and pinching finger 56 is again up so that they are essentially in position 68 at point in time t₁. The system at point t₃ is then in the same state it was at time t₁. Times t₄ and t₅ are merely repetitions of an additional cycle.

It is important to note that the speed with which the pumping fingers move toward the tubing during a cycle ideally is not constant. As the tubing is squeezed, equal increments of motion result in a displacement of progressively larger amounts of fluid. In other words, linear reciprocal motion of the finger against the tube as the tube becomes more compressed results in faster flow of fluid out from under the deformed tubing. To accommodate this, the ideal motion of pumping fingers 54 and 58 is such that each finger moves toward the tubing at a relatively rapid pace and then progressively slows as the tubing becomes more deformed. The benefit of such motion is a uniform rate of fluid flow forced by the squeezing action of the respective fingers.

Thus, the pumping mechanism having two cycles as described herein is a highly efficient apparatus for effecting fluid displacement. It also provides a linear, non-pulsatile flow of fluid which is desired in peristaltic pumping apparatus. In addition, this design thus allows the use of much smaller motors than would be necessary with either conventional linear or rotary peristaltic pumps. Since the size of the motor required generally reflects the peak rather than the average load encountered, this mechanism redistributes the load reflected so that the motor has a reduced peak. Further, the required occlusion is produced by two small pinching fingers that do not displace significant amounts of volume or distort the metering portion of the tubing. On the other hand, the pumping fingers, unlike conventional peristaltic fingers, never completely occlude the tubing or "crush" the tubing to produce undesired results.

It is also important to note that the present invention avoids waste of energy in linear and rotary peristaltic conventional pumps. The great bulk of such motive energy is typically consumed in heating the tubing through the high compressive and shear forces applied. This is because, as mentioned earlier, the fingers much not only pump but also occlude the tube. The present invention, however, separates the functions of pumping and occluding. Thus, the present invention has removed the need for repeated mashing and deformation of tubing to occlude it by the pumping aspect. in particular, the plastic set, or deformation, from repeated smashing of tubing does not affect the accuracy of the present apparatus. Any "set" around the area beneath the pinching fingers 52, 56 is much narrower since such fingers are much narrower and have the specific function of occluding the tube. However, the wider area under the wider pumping fingers 54 and 58 do not significantly experience the "set" phenomenon since they are not required to fully occlude the tubing. In other words, the pumping fingers 54, 58 have an upper and lower pumping finger limit as shown by the dimensions "b" and "c" in Figure 4. The pinching fingers 52 and 56, however, have a shorter motion to fully occlude the inside diameter "e" of the tube between finger heights "a" and the platen height "d".

Finally, with reference to Figure 6, there is shown an alternative embodiment representative of the present invention in which pumping fingers 54 and 58 are of identical size. The cam lobes 42 on shaft 28, however, are arranged such that the pumping fingers 54 and 58, being of the same size, are directed through a different amount of travel, respectively. In particular, it can be seen that while the limited travel of reciprocation of finger 58 is between elevations "b" and "c" as shown in Figure 6, the limited travel of reciprocation of finger 54 is between "b" and "f". The distance between "b" and "f" is a larger distance and thus there is a larger amount of travel. Provided such travel is accomplished during the same period of time, finger 54 thus displaces more fluid. By properly choosing the amount of travel and deformation of tubing involved, the amount of travel can thus be adjusted so that finger 54 squeezes tube 18 to displace a fluid volume that is approximately twice the fluid volume displaced by the squeezing movement of finger 58.

While the particular two-cycle peristaltic pump as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as defined in the appended claims.

## Claims

1. A device (10) for pumping fluid through a resilient tube (18) which comprises:
means (54,58) for alternately squeezing said tube (18) at a first location and at a second location, said squeezing means (54) being adapted to partially occlude said tube (18) at same first location to displace a fluid volume that is approximately twice the fluid volume displaced by said squeezing means (58) when said squeezing means (58) partially occludes said second location, said squeezing means (54,58) being moved towards said tube (18) at a progressively slower pace as said tube (18) becomes deformed to establish a substantially uniform rate of fluid flow through said tube (18); and
means (52,56) for alternately occluding said tube (18) upstream from said first location and between said first and second locations, said first location being squeezed as said tube (18) is occluded upstream from said first location, said occluding means (52,56) being synchronised with said squeezing means.

2. A device according to Claim 1 wherein said squeezing means comprises a first pumping finger (54) to urge against said tube (18) at said first location, said first pumping finger (54) being arranged for reciprocal movement between a withdrawn position and an extended position, and a second pumping finger (58) to urge against said tube (18) at said second location, said second pumping finger (58) being arranged for reciprocal movement between a withdrawn position and an extended position.

3. A device according to Claim 2 wherein said first pumping finger (54) is adapted to contact an area of said tube (18) that is approximately twice as large as the area of said tube (18) contacted by said second pumping finger (58).

4. A device according to Claim 2 wherein between their respective withdrawn and extended positions, said first pumping finger (54) travels a distance which is approximately twice the distance travelled by said second pumping finger (58).

5. A device according to Claim 2, 3 or 4 wherein said tube (18) remains patent at said first location and at said second location.

6. A device according to Claim 2, 3, 4 or 5 wherein the distance travelled by said first pumping finger (54) and said second pumping finger (58) between their respective withdrawn and extended positions are arranged to be less than the inner diameter of the tube (18).

7. A device according to any preceding claim wherein said second location is squeezed as said tube (18) is occluded between said first and second locations.

8. A device according to any preceding claim wherein said occluding means comprises a first pinching finger (52) for occluding said tube (18) upstream from said first location and a second pinching finger (56) for occluding said tube (18) between said first and second locations.

9. A method for pumping fluid through a resilient tube (18) which comprises the steps of:
alternately partially occluding said tube (18) at a first location and at a second location, said first location being partially occluded to displace approximately twice the fluid volume displaced when said second location is partially occluded, and wherein said partially occluding step is accomplished at a progressively slower pace as said tube (18) becomes deformed to establish a substantially uniform rate of fluid flow through said tube (18); and
alternately occluding said tube (18) upstream from said first location, and between said first and second locations.

10. A method according to Claim 9 wherein said step of squeezing said tube (18) at said first location is accomplished while said tube (18) is occluded upstream from said first location.

11. A method according to Claim 9 or 10 wherein said step of squeezing said tube (18) at said second location is accomplished while said tube (18) is occluded between said first and second locations.

12. A method according to Claim 9, 10 or 11 wherein said tube (18) remains patent at said first and second locations.

## Patentansprüche

1. Vorrichtung (10) zum Pumpen bzw. Fördern von Fluid durch einen elastischen Schlauch (18), umfassend:
Mittel (54, 58) zum abwechselnden Zusammendrücken des Schlauches (18) an einer ersten Stelle und einer zweiten Stelle, wobei das Zusammendrückmittel (54) den Schlauch (18) an der ersten Stelle teilweise zu okkludieren bzw. verschließen vermag, um ein Fluidvolumen zu verdrängen, das etwa das Doppelte des durch das Zusammendrückmittel (58) verdrängten Fluidvolumens, wenn das Zusammendrückmittel (58) (den Schlauch) an der zweiten Stelle teilweise verschließt, beträgt, wobei die Zusammendrückmittel (54, 58) im Laufe der Verformung des Schlauches (18) mit fortschreitend langsamerem Tempo gegen den Schlauch (18) verschoben werden, um eine im wesentlichen gleichmäßige Fluidströmungsmenge durch den Schlauch (18) einzustellen, und
Mittel (52, 56) zum abwechselnden Okkludieren bzw. Verschließen des Schlauches (18) stromauf der ersten Stelle sowie zwischen den ersten und zweiten Stellen, wobei die erste Stelle zusammengedrückt wird, wenn der Schlauch (18) stromauf der ersten Stelle okkludiert bzw. verschlossen ist, und die Okkludier- bzw. Verschließmittel (52, 56) mit den Zusammendrückmitteln synchronisiert sind.

2. Vorrichtung nach Anspruch 1, wobei die Zusammendrückmittel einen an der ersten Stelle gegen den Schlauch (18) andrückenden ersten Pumpfinger (54), der für geradlinige (Hin- und Her-)Bewegung zwischen einer zurückgezogenen Stellung und einer vorgeschobenen Stellung angeordnet ist, und einen an der zweiten Stelle gegen den Schlauch (18) andrückenden zweiten Pumpfinger (58), der für geradlinige (Hin- und Her-)Bewegung zwischen einer zurückgezogenen Stellung und einer vorgeschobenen Stellung angeordnet ist, umfassen.

3. Vorrichtung nach Anspruch 2, wobei der erste Pumpfinger (54) eine Fläche des Schlauches (18), die etwa doppelt so groß ist wie die vom zweiten Pumpfinger (58) kontaktierte Fläche des Schlauches (18), zu kontaktieren vermag.

4. Vorrichtung nach Anspruch 2, wobei der erste Pumpfinger (54) sich zwischen seinen jeweiligen zurückgezogenen und vorgeschobenen Stellungen über eine Strecke bewegt, die etwa das Doppelte der vom zweiten Pumpfinger (58) zurückgelegten Strecke beträgt.

5. Vorrichtung nach Anspruch 2, 3 oder 4, wobei der Schlauch (18) an der ersten Stelle und an der zweiten Stelle offen(stehend) (patent) bleibt.

6. Vorrichtung nach Anspruch 2, 3, 4 oder 5, wobei die vom ersten Pumpfinger (54) und vom zweiten Pumpfinger (58) zwischen ihren jeweiligen zurückgezogenen und vorgeschobenen Stellungen zurückgelegten Strecken so ausgelegt sind, daß sie kleiner sind als der Innendurchmesser des Schlauches (18).

7. Vorrichtung nach einem der vorangehenden Ansprüche, wobei die (an der) zweite(n) Stelle zusammengedrückt wird, während der Schlauch (18) zwischen den ersten und zweiten Stellen okkludiert bzw. verschlossen ist.

8. Vorrichtung nach einem der vorangehenden Ansprüche, wobei die Okkludier- bzw. Verschließmittel einen ersten Klemmfinger (52) zum Okkludieren bzw. Verschließen des Schlauches (18) stromauf der ersten Stelle und einen zweiten Klemmfinger (56) zum Okkludieren bzw. Verschließen des Schlauches (18) zwischen den ersten und zweiten Stellen umfassen.

9. Verfahren zum Pumpen bzw. Fördern von Fluid durch einen elastischen Schlauch (18), umfassend die folgenden Schritte:
teilweises Okkludieren bzw. Verschließen des Schlauches (18) an einer ersten Stelle und einer zweiten Stelle, wobei die erste Stelle teilweise okkludiert bzw. verschlossen wird, um etwa das Doppelte des Fluidvolumens zu verdrängen, das beim teilweisen Okkludieren bzw. Verschließen (an) der zweiten Stelle verdrängt wird, und wobei der Schritt des teilweisen Okkludierens bzw. Verschließens im Laufe der Verformung des Schlauches (18) mit fortschreitend langsamerem Tempo durchgeführt wird, um eine im wesentlichen gleichmäßige Fluidströmungsmenge durch den Schlauch (18) einzustellen, und
abwechselndes Okkludieren bzw. Verschließen des Schlauches (18) stromauf der ersten Stelle sowie zwischen den ersten und zweiten Stellen.

10. Verfahren nach Anspruch 9, wobei der Schritt des Zusammendrückens des Schlauches (18) an der ersten Stelle durchgeführt wird, während der Schlauch (18) stromauf der ersten Stelle okkludiert bzw. verschlossen ist.

11. Verfahren nach Anspruch 9 oder 10, wobei der Schritt des Zusammendrückens des Schlauches (18) an der zweiten Stelle durchgeführt wird, während der Schlauch (18) zwischen den ersten und zweiten Stellen okkludiert bzw. verschlossen ist.

12. Verfahren nach Anspruch 9, 10 oder 11, wobei der Schlauch (18) an den ersten und zweiten Stellen offen- (stehend) (patent) bleibt.

## Revendications

1. Un dispositif (10) destiné à pomper un fluide à travers un tube souple (18) qui comprend:
des moyens (54, 58) destinés à presser ledit tube (18) alternativement à un premier emplacement et à un second emplacement, lesdits moyens (54) destinés à presser étant adaptés pour obstruer partiellement ledit tube (18) à ce même premier emplacement afin de déplacer un volume de fluide qui soit environ deux fois le volume de fluide déplacé par lesdits moyens destinés à presser (58) lorsque lesdits moyens destinés à presser (58) obstruent partiellement ledit second emplacement, lesdits moyens destinés à presser (54, 58) étant déplacés vers ledit tube (18) à une allure progressivement plus lente à mesure que ledit tube (18) devient déformé afin d'instaurer un débit de fluide sensiblement uniforme à travers ledit tube (18) et
des moyens (52, 56) destinés à obstruer alternativement ledit tube (18) en amont dudit premier emplacement et entre lesdits premier et second emplacements, ledit premier emplacement étant pressé lorsque ledit tube (18) est obstrué en amont dudit premier emplacement, lesdits moyens destinés à obstruer (52, 56) étant synchronisés avec lesdits moyens destinés à presser.

2. Un dispositif selon la revendication 1, dans lequel lesdits moyens destinés à presser comprennent un premier ergot de pompage (54) pour appuyer sur ledit tube (18) audit premier emplacement, ledit premier ergot de pompage (54) étant disposé pour assurer un mouvement alternatif entre une position de retrait et une position avancée, ainsi qu'un second ergot (58) de pompage pour appuyer sur ledit tube (18) audit second emplacement, ledit second ergot de pompage (58) étant disposé pour assurer un mouvement alternatif entre une position de retrait et une position avancée.

3. Un dispositif selon la revendication 2, dans lequel ledit premier ergot de pompage (54) est adapté pour entrer en contact avec une zone dudit tube (18) qui soit environ deux fois plus importante que la zone dudit tube (18) sur laquelle appuie ledit second ergot de pompage (58).

4. Un dispositif selon la revendication 2, dans lequel, entre leurs positions respectives de retrait et avancée, ledit premier ergot de pompage (54) se déplace sur une distance qui est environ le double de la distance sur laquelle se déplace ledit second ergot de pompage (58).

5. Un dispositif selon la revendication 2, 3 ou 4, dans lequel ledit tube (18) reste ouvert audit premier emplacement et audit second emplacement.

6. Un dispositif selon la revendication 2, 3, 4 ou 5, dans lequel les distances sur lesquelles se déplacent ledit premier ergot de pompage (54) et ledit second ergot de pompage (58) entre leurs positions respectives de retrait et avancée sont conçues pour être inférieures au diamètre intérieur du tube (18).

7. Un dispositif selon l'une quelconque des revendications précédentes, dans lequel ledit second emplacement est pressé lorsque ledit tube (18) est obstrué entre lesdits premier et second emplacements.

8. Un dispositif selon l'une quelconque des revendications précédentes, dans lequel lesdits moyens destinés à obstruer comprennent un premier ergot de pincement (52) pour obstruer ledit tube (18) en amont dudit premier emplacement et un second ergot de pincement (56) pour obstruer ledit tube (18) entre les premier et second emplacements.

9. Une méthode pour pomper un fluide à travers un tube souple (18) qui comprend les étapes:
d'obstruction partielle dudit tube (18) alternativement au premier emplacement et au second emplacement, ledit premier emplacement étant partiellement obstrué afin d'assurer le déplacement d'environ deux fois le volume de fluide déplacé lorsque ledit second emplacement est obstrué partiellement, et dans laquelle ladite étape d'obstruction partielle est effectuée à une allure progressivement plus lente à mesure que ledit tube (18) devient déformé pour établir un débit de fluide sensiblement uniforme à travers ledit tube (18) et
d'obstruction alternative dudit tube (18) en amont dudit premier emplacement et entre lesdits premier et second emplacements.

10. Une méthode selon la revendication 9, dans laquelle ladite étape de resserrement dudit tube (18) audit premier emplacement est effectuée tandis que ledit tube (18) est obstrué en amont dudit premier emplacement.

11. Une méthode selon la revendication 9 ou 10, dans laquelle ladite étape de resserrement dudit tube (18) audit second emplacement est effectuée tandis que ledit tube (18) est obstrué entre lesdits premier et second emplacements.

12. Une méthode selon la revendication 9, 10 ou 11, dans laquelle ledit tube (18) reste ouvert auxdits premier et second emplacements.
